# EUROPEAN PATENT SPECIFICATION

(11) **EP 2 130 520 B1**
(45) Date of publication and mention of the grant of the patent: **27.06.2012**
(21) Application number: 09161303.4
(22) Date of filing: 27.05.2009
(51) Int. Cl.: A61F 2/46, G01K 11/12

(54) **Device for measuring the temperature of bone cement**
Vorrichtung zur Messung der Temperatur von Knochenzement
Dispositif pour mésurer la température du ciment d'os

(30) Priority: 05.06.2008 GB 0810252
(43) Date of publication of application: 09.12.2009
(73) Proprietor: DePuy International Limited, Beeston, Leeds LS11 8DT (GB)
(72) Inventor: Chandler, Matt, Leeds, LS11 8DT (GB)
(74) Representative: Belcher, Simon James

(56) References cited:
- EP-A- 0 380 867
- DE-U1- 29 607 832
- GB-A- 2 145 224
- US-A- 5 806 528

## Description

This invention relates to a device for use in the provision of bone cement to a patient, for example in an orthopaedic surgery procedure. The device might be used in preparing bone cement or in delivering bone cement or in both.

Bone cements that are used to provide fixation between bone tissue and an implanted prosthesis component (for example orthopaedic joint prosthesis components including spinal prostheses, and dental prostheses) are commonly provided by first and second materials which, when they react with one another, lead to the formation of a hard cement material. Examples of bone cement materials include those based on acrylate materials which can react by polymerising to form acrylate polymers. A bone cement composition can include acrylate polymer particles which react with monomer in the polymerisation reaction. A bone cement composition can also include other materials such as fillers, for example barium sulphate, zirconium dioxide, glass particles etc. A bone cement can be formed by mixing a liquid acrylate monomer with powders such as acrylate polymer particles and possibly barium sulphate, zirconium dioxide and/or glass particles. The resulting mixture has a paste or dough-like consistency. As is known, the components of the mixture react, involving polymerisation of the acrylate monomer and copolymerisation with the acrylate polymer particles. The viscosity of the cement composition increases during the reaction, resulting in a hard cement. The curing reaction of a bone cement material is generally exothermic.

It is known that surgical results can be optimised by ensuring that the cement is transferred from a mixing vessel to the prepared bone surface (for example, in a prepared bone cavity such as the intramedullary cavity in the femur or the humerus in the cases of a hip joint prosthesis or a shoulder joint prosthesis, or on a bone surface as in the case of a femur or a tibia in a knee joint prosthesis) where the prosthesis component is to be implanted when the cement is partially cured. The extent of the cure should exceed a minimum threshold so that the cement is not too fluid, facilitating handling of the cement and minimising the risk of the cement flowing undesirably after having been placed in contact with the prepared surface of the bone. As discussed below, the time taken to reach this stage in the cure reaction can be referred to as the End of Waiting Time. However, the extent of the cure should not exceed a maximum threshold, in order that subsequent introduction of the prosthesis component is not compromised, and in order that the cement should be able to penetrate the porous surface structure of the bone tissue. The time taken to reach this stage in the cure reaction can be referred to as the End of Working Time.

It is also known that surgical results can be optimised by ensuring that the prosthesis component is placed in contact with the bone cement (for example in a prepared bone cavity such as the intramedullary cavity in the femur or the humerus in the cases of a hip joint prosthesis or a shoulder joint prosthesis, or on a bone surface as in the case of a femur or a tibia in a knee joint prosthesis) when the cement is partially cured (with the extent of cure being greater than the extent of cure when the cement is placed on the prepared bone surface). The extent of the cure should exceed a minimum threshold so that the cement is not too fluid, which could mean that the cement would flow to an undesirable degree when the prosthesis component is deployed. However, the extent of the cure should not exceed a maximum threshold, in order that introduction of the prosthesis component and the formation of a bond to the component are not compromised.

The temperature of a bone cement material changes as the components of the material react, and this change in temperature can continue in the material after it has been placed in contact with a patient's bone. Monitoring the change in the temperature of the bone cement material can provide an indication of when the cement material has cured sufficiently for an implant to be secured against movement, for example to allow a surgeon to release the implant and to proceed with another stage of the surgical procedure. This is sometimes referred to as the Final Setting Time.

It is common for a surgeon to determine the extent of cure by feel, involving kneading the cement as it cures and relying on judgement to assess whether the extent of cure of the cement has reached such a level that it is appropriate to transfer the cement to the prepared bone surface, and to such a level that it is appropriate subsequently to introduce the prosthesis component to the cement. Features of the cement which characterise its extent of cure include viscosity (or firmness), tackiness, and smoothness ("grittiness"). Assessment of these features can be affected by environmental conditions. The speed of cure is affected by the temperature of the cement components when they are mixed and on ambient temperature. A factor such as the perceived tackiness can be affected by temperature and humidity.

Relying on subjective techniques such as feel to determine the extent of cure has the disadvantage that it is not always reliable, and it can be difficult to train new users of these techniques. Furthermore, the nature of the cure reaction is such that it can be affected by variations in conditions, especially temperature. Notably, variations in the temperature of the surgeon's fingers as he kneads a sample of a bone cement can lead to variations in the extent of cure of that sample, relative to the extent of cure of the remainder of the cement which is to be used in the procedure. Also, variations in temperature and humidity will affect the perception of the tackiness of the cement.

EP-A-380867 discloses a bone cement mixing system which uses a cartridge mixing device. The extent of cure of the cement can be monitored by placing a small quantity of the cement in a container whose wall is formed by a temperature sensitive tape.

GB-A-2145224 discloses a flexible tape which comprises a transparent flexible sheet having a thin layer of a thermochromic liquid crystal applied to it. It can be used to monitor changes in the temperature of an animal due to infection.

The present invention provides a kit which comprises (a) a quantity of a bone cement, and (b) a device for use in the provision of the bone cement to a patient, as defined in claim 1.

The invention also provides a method of monitoring the extent of cure of a bone cement, as defined in claim 6.

Preferably, the method includes the step of measuring the quantity of the cement which is wrapped in the film to ensure that it is within a preset range. The quantity can be measured volumetrically or by weight. The thermal trend during cure of the cement can vary depending on the quantity of cement that is under observation. It can therefore be preferred to measure the quantity that is used in the method to ensure that the extent of cure when the thermochromic material changes colour is understood accurately. An example of a suitable quantity of cement is about 20 g.

Preferably, the quantity of cement is shaped so that it is relatively compact, for example generally rounded, especially approximately spherical.

The kit of the invention, which comprises components of a bone cement material and a film in which the mixed bone cement material can be wrapped, can include multiple sheets of the film which are sized appropriately to wrap around the predetermined quantity of the cement. The sheets might be approximately square. The sheets might be approximately circular.

The device might include a closure to retain the film wrapped around the bone cement. The closure might gather the film into a bundle, for example as a clip or a tie-wrap. The closure might serve to adhere the film edge to an underlying layer of the film, for example a quantity of adhesive backed tape.

The film, by virtue of its thermochromic material, can provide an indication of the extent of cure of a bone cement material. The indication will involve a change in the colour of the film, caused by exposure of the film to a change in temperature of the bone cement material as it cures. The temperature at which the change in the colour of the thermochromic material takes place can be selected by appropriate selection of the material. The thermochromic material can be selected to indicate a colour change when the temperature of the bone cement material is characteristic of, for example, the End of Waiting Time or the End of Working Time or the Final Setting Time of the cement material. A plurality of different thermochromic materials can be used to indicate temperatures which are characteristic of different stages in the cure of a cement material.

Thermochromic materials which can be used in the device of the invention can change between two colours, or between a coloured condition and colourless. A thermochromic material can be characterised by an activation temperature, which is the temperature at which the material has reached its final colour (or clear) state. The colour change has been found to take place when the sensed temperature increases towards the activation temperature over a small range of temperatures extending from about 4°C below to the activation temperature to the activation temperature.

Suitable materials for use as the thermochromic material are available from B&H Colour Change Limited of London GB-SW18 2RU.

The film can have first and second regions, in which the film is loaded with a first thermochromic material in the first region and is loaded with a second thermochromic material in the second region, and in which the temperature which is indicated by the first thermochromic material is different from the temperature which is indicated by the second thermochromic material.

Polymers which can be used in the indicator component can include polyolefins, polyamides, polyesters. Examples of preferred polymers include ethylene polymers and propylene polymers, especially low density polyethylene.

Preferably, the thickness of the film is not more than about 500 µm, more preferably not more than about 200 µm, especially not more than about 100 µm, for example not more than about 50 µm, or not more than about 30 µm. Preferably, the thickness of the film is at least about 2 µm, more preferably at least about 5 µm, for example at least about 8 µm. The thickness of the film should be selected so that the film is flexible, allowing it to be wrapped around a quantity of the cement. The thickness of the film should be selected so that it is sufficiently tough so that it is not susceptible to damage as it is wrapped around the cement and then exposed to elevated temperature when the cement cures.

The film can be formed by a process which includes steps such as extrusion and drawing. It is desirable that the processing steps be selected to ensure that the film is flexible so that it can be wrapped around a quantity of the cement so as to conform to the cement tightly. Techniques for forming thin flexible films are well known, for example as used to prepare thin films which are used to wrap foodstuffs.

The thermochromic material can be combined with the polymer of the film as a powder. Particles of the thermochromic material can include a thermochromic dye material which is encapsulated in a polymeric carrier (for example comprising a low density polyethylene). For example, a thermochromic material can be provided in the form of microcapsules which contain crystal violet lactone, a weak acid, and a dissociable salt dissolved in a nonpolar or slightly polar solvent liquid crystal solvent such as dodecanol or another suitable liquid crystal solvent. When the mixture is a solid, the dye exists in its lactone leuco form. However, when the liquid crystal solvent melts, the salt dissociates, the pH inside the microcapsule lowers (making protons readily available), the dye becomes protonated, and the lactone ring opens causing its absorption spectrum to shift, absorbing in the visible spectrum, such as a deeply violet colour for crystal violet lactone.

Suitable thermochromic dyes can be based on mixtures of leucodyes with suitable other chemicals, which display a colour change (usually between a colourless leuco form and the coloured form of the dye) dependent on the temperature. The dyes can be applied on the bone cement directly.

Examples of thermochromic materials which can be used in the device of the invention include spirolactones, fluorans, spiropyrans, and fulgides. Weak acids that can be used as proton donors include bisphenol A, parabens, 1,2,3-triazole derivatives, and 4-hydroxy-coumarin. These weak acids can function as a proton donor to cause a dye molecule to change between its leuco form and its protonated coloured form. Stronger Brönsted acids (better proton donors) can also be used but they tend to make the colour change irreversible. Other thermosensitive dyes that can be used include an oxazine-based leuco thermosensitive dye (such as that sold under the trade mark CSB-12 by Hodogaya Chemicals Co), a spiropyran-based leuco thermosensitive dye (such as that sold under the trade mark CSR-13 by Hodogaya Chemicals Co), a quinoline-based thermosensitive dye (such as that sold under the trade mark CSY-13 by Hodogaya Chemicals Co) and the like.

A plurality of thermosensitive dyes are known and are available commercially. The thermosensitive dyes are not particularly limited, but it is desired that dyes that are not toxic are used. A plurality of thermosensitive dyes are available that change colours at a variety of temperatures. Suitable thermochromic dyes are known which activate at temperatures in the range of 21 to 51 °C and which are available from SICPA Securink Corporation of Springfield, VA. These dyes include 744020TC (thermochromic blue), 744010TC (thermochromic turquoise), 744027TC (thermochromic yellow), 734010TC (thermochromic rose), 724010TC (thermochromic orange), 754027TC (thermochromic green). There are also thermochromic dyes which lose colour when heated, so that they change from a colour towards clear. These dyes include 178002TC (black/clear) which is active at 27 to 36°C. Compounds which are active at 22 to 31°C include 128001TC (orange/clear), 1384175TC (rose/clear), 150015TC (green/clear), 148003TC (blue/clear), 17800TC (black/clear), 14001TCBR (blue/red) and 128001TCY (orange/yellow). Compounds which are active from 24 to 33°C include 118000TC (yellow/clear), 128002TC (orange/clear), 138103TC (vermillion/clear), 15002TC (green/clear), 14001TC (blue/clear), 14000TCBR (blue/red) and 128001TCY (orange/yellow). Compounds which are active at 24 to 33°C include 11800TC (yellow/clear), 128002TC (orange/clear), 138103TC (vermillion/clear), 15002TC (green/clear), 14001TC (blue/clear), 14000TCBR (blue/red) and 128002TC (orange/yellow). Compounds which are active at 32 to 41°C include 13001TC (rose/clear), 148002TC (blue/clear), 178001TC (black/clear) and 178002TCBR (blue/red). The compound should be non-toxic. It is an advantage of the device of the present invention that the compound does not form part of a component or a composition which is implanted in a patient. This can reduce the risk of an adverse reaction as a result of contact with a patient's tissue or body fluid.

It will often be preferred that a change in the colour of the film be detected by visually inspecting the colour change. Alternatively, a spectrophotometer or some other optical sensor instrument can be used to detect the colour change. It can however be preferred to use an optical sensor, for example to provide greater precision, or to differentiate between small changes in colour. Moreover, using an instrument that can detect colour change allows one to find the optimum extent of cure when tints of various colours are detected (i.e., small changes on the pantone scale).

The concentration of the thermochromic material in the polymer of the indicator component will be selected to provide an adequately visible colour change response. The composition of the material of the indicator component should also take into account characteristics such as the desired physical properties of the component. The selection of an appropriate composition will be straightforward for the skilled person.

## Claims

1. A kit which comprises (a) a quantity of a bone cement, and (b) a device for use in the provision of the bone cement to a patient, which comprises a polymer which is loaded with a thermochromic material, **characterised in that** the device comprises a flexible polymeric film in which the polymer of the film is loaded with the thermochromic material, the film being suitable for wrapping around the quantity of the mixed cement so that the film is in contact with and tightly conforms to the cement.

2. A kit as claimed in claim 1, in which the polymer of the film comprises a polyolefin.

3. A kit as claimed in claim 1, in which the film has first and second regions, in which the film is loaded with a first thermochromic material in the first region and is loaded with a second thermochromic material in the second region, and in which the temperature which is indicated by the first thermochromic material is different from the temperature which is indicated by the second thermochromic material.

4. A kit as claimed in claim 1, in which the thickness of the film is not more than about 500 µm.

5. A kit as claimed in claim 1, in which the thickness of the film is at least about 2 µm.

6. A method of monitoring the extent of cure of a bone cement, which comprises:
a. wrapping a quantity of the mixed cement in a flexible polymeric film so that the film is in contact with and tightly conforms to the cement, in which the polymer of the film is loaded with a thermochromic material,
b. monitoring the film to identify a change in the colour of the film, indicating that the bone cement which is wrapped in the film has reached a predetermined temperature.

7. A method as claimed in claim 6, which includes the step of measuring the quantity of the cement which is wrapped in the film to ensure that it is within a preset range.

## Patentansprüche

1. Kit, der (a) eine Menge eines Knochenzements und (b) eine Vorrichtung zur Verwendung bei der Bereitstellung des Knochenzements für einen Patienten, die ein Polymer umfasst, das mit einem thermochromen Material beladen ist, umfasst, **dadurch gekennzeichnet, dass** die Vorrichtung einen flexiblen Polymerfilm umfasst, wobei das Polymer des Films mit dem thermochromen Material beladen ist, wobei der Film geeignet ist zum Herumwickeln um die Menge des gemischten Zements, sodass der Film in Kontakt mit dem Zement steht und eng an diesem anliegt.

2. Kit nach Anspruch 1, wobei das Polymer des Films ein Polyolefin umfasst.

3. Kit nach Anspruch 1, wobei der Film erste und zweite Regionen aufweist, wobei der Film mit einem ersten thermochromen Material in der ersten Region beladen ist und mit einem zweiten thermochromen Material in der zweiten Region beladen ist und wobei die Temperatur, die von dem ersten thermochromen Material angezeigt wird, verschieden ist von der Temperatur, die von dem zweiten thermochromen Material angezeigt wird.

4. Kit nach Anspruch 1, wobei die Dicke des Films nicht größer ist als etwa 500 µm.

5. Kit nach Anspruch 1, wobei die Dicke des Films wenigstens etwa 2 µm beträgt.

6. Verfahren zur Überwachung des Ausmaßes der Aushärtung eines Knochenzements, welches umfasst:
a. Einwickeln einer Menge des gemischten Zementes in einem flexiblen Polymerfilm, sodass der Film in Kontakt mit dem Zement steht und eng an diesem anliegt, wobei das Polymer des Films mit einem thermochromen Material beladen ist,
b. Überwachen des Films, um eine Veränderung in der Farbe des Films zu identifizieren, die anzeigt, dass der Knochenzement, der im Film eingewickelt ist, eine vorbestimmte Temperatur erreicht hat.

7. Verfahren nach Anspruch 6, das den Schritt des Messens der Menge des Zementes einschließt, die im Film eingewickelt ist, um sicherzustellen, dass sie innerhalb eines vorbestimmten Bereiches liegt.

## Revendications

1. Kit comprenant : (a) une quantité de ciment osseux, et (b) un dispositif destiné à délivrer le ciment osseux chez un patient, qui comprend un polymère qui est chargé avec un matériau thermochromique, **caractérisé en ce que** le dispositif comprend un film polymère souple dans lequel le polymère du film est chargé avec le matériau thermochromique, le film étant approprié pour s'envelopper autour de la quantité de ciment mélangé de telle sorte que le film soit en contact avec le ciment et se conforme étroitement à celui-ci.

2. Kit selon la revendication 1, dans lequel le polymère du film comprend une polyoléfine.

3. Kit selon la revendication 1, dans lequel le film présente des première et seconde régions, dans lequel le film est chargé avec un premier matériau thermochromique dans la première région et est chargé avec un second matériau thermochromique dans la seconde région, et dans lequel la température qui est indiquée par le premier matériau thermochromique est différente de la température qui est indiquée par le second matériau thermochromique.

4. Kit selon la revendication 1, dans lequel l'épaisseur du film n'est pas supérieure à 500 µm environ.

5. Kit selon la revendication 1, dans lequel l'épaisseur du film est au moins égale à 2 µm environ.

6. Procédé destiné à surveiller le degré de séchage d'un ciment osseux, qui comprend les étapes consistant à :
a) envelopper une quantité de ciment mélangé dans un film polymère souple de telle sorte que le film soit en contact avec le ciment et se conforme étroitement à celui-ci, dans lequel le polymère du film est chargé avec un matériau thermochromique ;
b) surveiller le film de façon à identifier un changement de la couleur du film, qui indique que le ciment osseux qui est enveloppé dans le film a atteint une température prédéterminée.

7. Procédé selon la revendication 6, qui comprend une étape consistant à mesurer la quantité de ciment qui est enveloppé dans le film de façon à s'assurer qu'elle se situe à l'intérieur d'une plage préréglée.
